(19) Europäisches Patentamt European Patent Office Office européen des brevets

(11) **EP 3 970 615 B1**

(12) **EUROPEAN PATENT SPECIFICATION**

(45) Date of publication and mention of the grant of the patent:
**08.01.2025 Bulletin 2025/02**

(21) Application number: **21197579.2**

(22) Date of filing: **18.09.2021**

(51) International Patent Classification (IPC):
**A61B 5/308** (2021.01)     **A61B 5/352** (2021.01)
**A61B 5/00** (2006.01)

(52) Cooperative Patent Classification (CPC):
**A61B 5/308; A61B 5/352; A61B 5/7203;**
A61B 5/358

(54) **METHOD FOR DETECTING QRS COMPLEX OF ELECTROCARDIOGRAM SIGNAL**

VERFAHREN ZUR DETEKTION EINES QRS-KOMPLEXES EINES ELEKTROKARDIOGRAMMSIGNALS

PROCÉDÉ POUR DÉTECTER LE COMPLEXE QRS D'UN SIGNAL D'ÉLECTROCARDIOGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.09.2020 PL 43539420**

(43) Date of publication of application:
**23.03.2022 Bulletin 2022/12**

(73) Proprietor: **Akademia Gorniczo-Hutnicza im. Stanislawa Staszica w Krakowie 30-059 Krakow (PL)**

(72) Inventors:
- **REKLEWSKI, Wojciech**
  **02-493 Warszawa (PL)**
- **HERYAN, Katarzyna**
  **30-619 Krakow (PL)**
- **AUGUSTYNIAK, Piotr**
  **31-457 Krakow (PL)**
- **MISKOWICZ, Marek**
  **31-621 Krakow (PL)**

(74) Representative: **Belz, Anna ul. Pólnocna 6/24 20-064 Lublin (PL)**

(56) References cited:
**KR-A- 20160 107 390**

- **CHEN H C ET AL: "A moving average based filtering system with its application to real-time QRS detection", COMPUTERS IN CARDIOLOGY 2003. THESSALONIKI, GREECE, SEPT. 21 - 24, 2003; [COMPUTERS IN CARDIOLOGY], NEW YORK, NY : IEEE, US, vol. VOL. 30, 21 September 2003 (2003-09-21), pages 585 - 588, XP010698972, ISBN: 978-0-7803-8170-4, DOI: 10.1109/CIC.2003.1291223**
- **S-W CHEN: "A nonlinear trimmed moving averaging-based system with its application to real-time QRS beat classification", JOURNAL OF MEDICAL ENGINEERING & TECHNOLOGY, INFORMA HEALTHCARE, GB, vol. 31, no. 6, 1 November 2007 (2007-11-01), pages 443 - 449, XP008175381, ISSN: 0309-1902, DOI: 10.1080/ 03091900701234267**
- **JALALEDDINE S ET AL: "AMBULATORY ECG WAVE DETECTION FOR AUTOMATED ANALYSIS: A REVIEW", ISA TRANSACTIONS, INSTRUMENT SOCIETY OF AMERICA. PITTSBURGH, US, vol. 26, no. 4, 1 January 1987 (1987-01-01), pages 33 - 43, XP000046086, ISSN: 0019-0578**
- **RAHUL JAGDEEP ET AL: "Dynamic thresholding based efficient QRS complex detection with low computational overhead", BIOMEDICAL SIGNAL PROCESSING AND CONTROL, ELSEVIER, AMSTERDAM, NL, vol. 67, 9 March 2021 (2021-03-09), XP086536385, ISSN: 1746-8094, [retrieved on 20210309], DOI: 10.1016/ J.BSPC.2021.102519**

## Description

The subject of the invention is a method for detecting the QRS complex of an electrocardiogram (ECG) signal as defined in the appended claims.

A device for monitoring the ECG signal, consisting of technical means for measuring the pulse, filters and amplifiers for noise elimination and amplification of the diagnostically useful signal, and a computer programmed to suppress frequency components below 15 Hz and above 25 Hz and amplify the R wave in the QRS signal is known from the patent description US5738104.

A heart rate monitor for calculating heart rate based upon ECG signals. The monitor preferably utilizes 3 electrodes to pick up ECG signals and a differential amplifier to cancel common mode signals in the output of the electrode. An analog bandpass filter comprised a low pass and high pass filter in series each with different rolloffs filters out low and high frequency components. The signals are digitized and digital filtering to remove power line hum and remnants of low and high frequency noise is performed. Then the ECG signals are digitally enhanced by differentiating and squaring the results of the differentiator then being averaged in a moving average computation so as to generate enhanced digital data. The enhanced digital data is then processed to learn the ECG characteristics, and a heart rate arbitrator processes the incoming signals to select out actual ECG complexes from EMG noise and other noise. The ECG isolation process is done using rules of reason and the learned characteristics of the ECG signal.

A device and method is known from US5188116 patent for electrocardiographic tests, the device of which is a system for the analysis of electrocardiographic activity for the detection of ischemic heart disease, consisting of tools for detecting a multiplicity of periodic electrocardiographic signals, a memory unit storing these signals, a microprocessor capable of calculating collective cycles for each signal, detecting and storing signal amplitude characteristics, as well as determining the variance of the signals and modifying them, furthermore from technical means for determining the degree of coronary artery disease. The patent also describes a method of analyzing electrocardiographic activity for the detection of ischemic heart disease, including collecting and storing electrocardiographic signals, establishing a cumulative cycle for each signal, determining the variance of each signal and the total variability of all signals, and then determining the size of the coronary heart disease therefrom.

There is known from the US patent US9414761 a method of processing electrocardiographic signals comprising filtering the ECG signal by passing the ECG signal through at least one first low-pass filter and a high-pass filter, obtaining a processed ECG signal by passing the trailing averages of the ECG signal envelope through a second lowpass filter, identifying a search region in the processed ECG signal, the search region being the time interval between two local peaks of the processed ECG signal, and identifying the maximum amplitude pulse within the search region in processed ECG signal that is considered to be the R wave in the QRS complex of the ECG signal.

A method is known from the article "A Real-Time QRS Detection Algorithm", IEEE Transactions on Biomedical Engineering, vol. 32, no. 3, 1985, by J. Pan, W.J. Tompkins for detecting the QRS complex of the electrocardiogram (ECG) signal, including the reception of the ECG signal obtained by means of an ECG recorder, filtering the ECG signal by passing the ECG signal through a low-pass filter and then a high-pass filter with parameters selected in such a way that both filters jointly create a band-pass filter, extracting information about the slope of the QRS complex of the ECG signal by passing the ECG signal after filtering through the differential system, then emphasizing information about the slopes of the QRS complex by squaring the resulting signal, then calculating average value of the resulting signal for a time interval of a fixed length until the threshold has been exceeded, which is considered equivalent to detecting the R-wave in the QRS complex, resulting in an adaptation of the threshold, and a re-comparison of the signal values after a time interval of 200 ms.

A method known from the article "A moving average based filtering system with its application to real-time QRS detection", Computers in Cardiology, 2003, pp. 585-588, by Chen, H. C., and Chen, S. W. comprises the calculation of a moving average of an input ECG signal, which its further subtracted from the input ECG signal. Next, the resultant signal is squared, and the moving average of the squared signal is calculated, which is an input to a decision block. The input signal to the decision block is compared to the threshold calculated as THRESHOLD(n) = alfa * gamma * PEAK + (1-alfa)THRESHOLD(n-1), while PEAK is a new local max in the input to the decision block, alfa and gamma are coefficients between 0 and 1, and n-1 is an index of the previous QRS detection cycle. The QRS is detected only when the peak level of the feature signal exceeds the threshold.

A device known from the patent application KR 2016 0107390 comprises an ECG signal detection module wherein an ECG signal is subject to a low pass filter and a high pass filter, as well as a control unit that differentiates an output signal of the pass filter. The controller sets a window of 80ms and averages an absolute value of the differentiated signal in a section within the set window to repeatedly obtain a peak value at a predetermined period of time.

The following terms have been introduced in current description as below:

(1) ECG_MODULE - an electrocardiogram (ECG) signal measurement module,

(2) ABS_DIFF_SHORT_MODULE - a module that determines the ABS_DIFF_SHORT signal, which is a difference between a current instantaneous value of the ECG signal and the average SHORT_AVG value of the ECG signal calculated for a T_SHORT time interval of a fixed length,

(3) ABS_DIFF_LONG_MODULE - a module that determines the ABS_DIFF_LONG signal, which is a difference between a current instantaneous value of the ECG signal and the average LONG_AVG value of the ECG signal calculated for a T_LONG time interval of a fixed length,

(4) COMP - a comparator,

(5) PEAK_DETECTOR - a detector of the maximum value of the ABS_DIFF_LONG signal during the SEARCHING_WINDOW time window for searching of a R wave in the QRS complex,

(6) PULSE GENERATOR - a pulse generator which, when a triggering signal arrives, generates a pulse of a fixed duration equal to the SEARCHING_WINDOW time window for searching the R wave in the QRS complex of the ECG signal,

(7) TH_MODULE - a module that determines the threshold TH value for the ABS_DIFF_SHORT signal, used to determine the beginning of the SEARCHING_WINDOW time window for searching the R wave in the QRS complex of the ECG signal,

(8) R_AMPLITUDE_MEMORY - a memory module for storing information about the amplitudes of the maximum values of the LONG_ABS_DIFF signal in subsequent cycles of the ECG signal, which are considered to be the amplitudes of the R waves in subsequent QRS complexes of the ECG signal,

(9) R _TIMESTAMP_MEMORY - a memory module for storing information about the times of the maximum LONG_ABS_DIFF signal values in the subsequent cycles of the ECG signal, which are considered to be the times of the R waves in subsequent QRS complexes of the ECG signal.

According to the invention, a method for detecting the QRS complex of the electrocardiogram (ECG) signal consisting in a reception of the ECG signal obtained by a ECG signal measurement module monitoring an electrical activity of a patient's heart is characterized in that a COMP comparator monitors the ABS_DIFF_SHORT signal generated at the ABS_DIFF_SHORT_MODULE output, said signal being a difference of a current instantaneous value of the ECG signal provided by the ECG_MODULE measurement module, and an average SHORT_AVG value of the ECG signal calculated for a T_SHORT time segment of a fixed length. Then, using the COMP comparator, a moment when the ABS_DIFF_SHORT signal reaches a threshold TH value, previously determined by the TH_MODULE module, is detected, thereafter, as soon as the ABS_DIFF_SHORT signal reaches a predetermined threshold TH value, the timing of the SEARCHING_WINDOW time window of a predetermined length to search for a R wave in the QRS complex of the ECG signal is started by the impulse generator PULSE GENERATOR. In the next step, using the PEAK_DETECTOR detection module, the maximum value of the ABS_DIFF_LONG signal produced on the output of the ABS_DIFF_LONG_MODULE module, whereas ABS_DIFF_LONG signal is a difference of a current instantaneous value of the ECG signal and an average LONG_AVG value of the ECG signal calculated per fixed-length T_LONG time interval, is recorded during the SEARCHING_WINDOW time window, and at the same time, the time of occurrence of the detected maximum value of the ABS_DIFF_LONG signal during the SEARCHING_WINDOW time window is recorded with the PEAK_DETECTOR detection module. After these operations, the maximum value of the ABS_DIFF_LONG signal detected by the PEAK_DETECTOR detection module, which is considered to be the amplitude of the R wave in the QRS complex, is stored by means of the memory module R_AMPLITUDE_MEMORY.

Then, the time of the occurrence of the maximum value of the ABS_DIFF_LONG signal detected by the PEAK_DETECTOR detection module, which is considered to be the time of the occurrence of the R wave in the QRS complex, is stored by means of the R_TIMESTAMP_MEMORY memory module.

In the next step, using the TH_MODULE module, a new threshold TH value is determined, which is used to trigger the beginning of the SEARCHING_WINDOW time window in the next cycle of the ECG signal with the PULSE GENERATOR impulse generator. The new TH value threshold is determined on the basis of the maximum values of the ABS_DIFF_LONG signal in the previous ECG signal cycles detected by the PEAK_DETECTOR detection module and stored with the R_AMPLITUDE_MEMORY memory module. Then, the monitoring of the ABS_DIFF_SHORT signal generated at the output of the ABS_DIFF_SHORT_MODULE module on the basis of the ECG signal provided by the ECG signal measuring module is resumed by means of the COMP comparator. After that, the entire cycle described above is repeated as many times as required.

The T_SHORT time period, used to determine the SHORT_AVG average value of the ECG signal, is not shorter than 40 ms and also not longer than 100 ms, while the T_LONG time period, used to determine the LONG_AVG average value of the ECG signal, is not shorter than 150 ms and also not longer than 400 ms.

The TH value threshold for the ABS_DIFF_SHORT signal, which is the difference between the current instantaneous value of the ECG signal provided by the ECG_MODULE measurement module, and the average SHORT_AVG value of the ECG signal calculated for the T_SHORT time interval of fixed length, in a given detection cycle of the QRS complex, is determined by the TH_MODULE module. This is done on the basis of the TH threshold value determined by the TH_MODULE module in the previous QRS complex detection cycle and recorded with the PEAK_DETECTOR detection module, and the R-wave amplitude stored in the R_AMPLITUDE_MEMORY memory module in the QRS complex recorded in the current QRS complex detection cycle. The TH threshold in a given QRS complex detection cycle is the sum of the product of the TH threshold value in the previous QRS detection cycle and the scaling factor less than unity and the product of the R-wave amplitude recorded in the current QRS complex detection cycle, one minus the scaling factor, and the weighting factor also less than one. The threshold TH value before the first detection cycle of the QRS complex is determined by the TH_MODULE module in a form of the product of the weighting factor that is less than one and the average value of the ECG signal calculated for a time interval of one second.

The method for detecting the QRS complex in the electrocardiogram signal by referring, through the subtraction of the current instantaneous value of the ECG signal from its average values, calculated respectively for the T_SHORT and T_LONG time segments is immune to noise and disturbances occurring during the measurement of the electrocardiogram signal. In addition, thanks to a continuous adjustment of the TH threshold value - by means of the TH_MODULE module - used to determine the beginning of the SEARCHING_WINDOW time window for searching the R wave in the QRS complex, to the TH threshold value determined in the previous QRS detection cycle and to the amplitude of the R wave in the current QRS detection cycle, the R wave detection sensitivity to transient fluctuations in the maximum range of changes of the electrocardiogram signal that may occur during the measurement of the ECG signal, is reduced.

The electrocardiogram (ECG) signal, representing the electrical activity of the patient's heart, is received with the ECG_MODULE measurement module via electrodes attached to the patient's body. The ECG signal can be reduced to a sequence of positive and negative deviations (waves) from the isoelectric line, which corresponds to the periods of time during which no heart beats are detected. The group of the largest waves, called the QRS complex, consists of a negative deflection (Q wave), a positive deflection (R wave), and another negative deflection (S wave). The R wave has usually the highest amplitude in the QRS complex. The detection of the QRS complex is often reduced to the detection of the R wave. Statistics of the intervals between the R waves and the amplitudes of the R waves are important diagnostic information, used in medicine, among other things, to diagnose the work of the heart.

According to the invention, when detecting each QRS complex in the ECG signal with the ABS_DIFF_SHORT_MODULE module, the ABS_DIFF_SHORT signal is determined on the basis of the ECG signal, which is the difference of the current instantaneous value of the ECG signal and the average SHORT_AVG value of the ECG signal calculated for the T_SHORT time interval of 55 ms. At the same time, the ABS_DIFF_LONG_MODULE module determines the ABS_DIFF_LONG signal, which is the difference between the current instantaneous value of the ECG signal and the average LONG_AVG value of the ECG signal calculated for the T_LONG time interval of 277 ms. Monitoring of the ABS_DIFF_SHORT signal is aimed at detecting the beginning of the rising edge of the ECG signal preceding the occurrence of the R wave in the QRS complex, and subtracting the current value of the ECG signal from the average SHORT_AVG value is aimed at filtering out any noise and disturbances present in the ECG signal received by the ECG_MODULE measuring module. The lengths of the time segments, T_SHORT and T_LONG, respectively, result from the dynamics of the human heart.

The SHORT_ABS_DIFF signal available at the output of the ABS_DIFF_SHORT_MODULE module is continuously compared by means of a COMP comparator with a predetermined TH value threshold determined by the TH_MODULE module. The detection of the SHORT_ABS_DIFF signal reaching a predetermined TH threshold value is taken to be the start of the rising edge of the ECG signal preceding the occurrence of the R wave in the QRS complex. When it is detected that the SHORT_ABS_DIFF signal has reached the predetermined TH threshold, the COMP comparator, with the help of an appropriate signal on its output, starts generating a 200 ms long pulse using the PULSE GENERATOR pulse generator, which determines the SEARCHING_WINDOW time window, during which the R wave is searched for in the QRS complex of the ECG signal. The pulse generated at the output of the PULSE GENERATOR pulse generator activates the PEAK_DETECTOR module with its active logic level, which records the maximum value of the LONG_ABS_DIFF signal generated at the output of the ABS_DIFF_LONG_MODULE module during the SEARCHING_WINDOW time window. Subtracting the current value of the ECG signal from the average value of LONG_AVG is designed to filter out possible noise and disturbances in the ECG signal received by the ECG_MODULE measurement module.

The PEAK_DETECTOR module also records a moment when the detected maximum value of the ABS_DIFF_LONG signal which occurred during the SEARCHING_WINDOW time window.

The maximum value of the ABS_DIFF_LONG signal produced at the output of the ABS_DIFF_LONG_MODULE module during the SEARCHING_WINDOW time window is considered to be the R-

wave amplitude in the QRS complex of the ECG signal. In turn, the moment of occurrence of the detected maximum value of the LONG_ABS_DIFF signal during the SEARCHING_WINDOW time window is considered to be the moment of the R wave occurrence in the QRS complex of the ECG signal. Then, when the trailing edge of the pulse generated at the output of the pulse generator PULSE GENERATOR occurs, the maximum value of the LONG_ABS_DIFF signal previously detected by PEAK_DETECTOR module is recorded by means of the module R_AMPLITUDE_MEMORY, and the moment of the maximum signal value of the LONG_ABS_DIFF signal is recorded by means of the R_AMPLITUDE_MEMORY module respectively.

Then, by means of the TH_MODULE module, a new threshold TH value is determined, which serves for detection of the beginning of the SEARCHING_WINDOW time window by means of a PULSE GENERATOR pulse generator. The new TH value threshold is determined on the basis of the threshold TH value determined by the TH_MODULE module in the previous QRS detection cycle recorded with the PEAK_DETECTOR detection module and the R-wave amplitude stored in the R_AMPLITUDE_MEMORY memory module in the QRS complex in the current QRS complex detection cycle in such a way that the TH value threshold in a given QRS complex detection cycle is the sum of the product of the TH threshold value determined in the previous QRS complex detection cycle and the scaling factor less than one and the product of the R wave amplitude recorded in the current QRS complex detection cycle, one minus the scaling factor, and a weight factor also smaller than one, wherein the TH threshold value before the first detection cycle of the QRS complex is determined by the TH_MODULE module in the form of a the product of less than one weighting factor and the maximum value of the ECG signal over a time interval of one second.

The above-mentioned rule for determining a new TH threshold value using the TH_MODULE module corresponds to the following mathematical formula:

$$\mathrm{TH_i} = \gamma\mathrm{TH_{i-1}} + \alpha(1 - \gamma)\mathrm{R_i}$$

where $\gamma$ <1 means the scaling factor, $\alpha$ <1 means the weighting factor, $\mathrm{TH_i}$ means the TH threshold value determined in the current QRS complex detection cycle, $\mathrm{TH_{i-1}}$ means the TH threshold value determined in the previous QRS complex detection cycle, $\mathrm{R_i}$ means the R-wave amplitude recorded in the current QRS complex detection cycle, and

$$\mathrm{TH_0} = \alpha\mathrm{R_0}$$

where $\mathrm{TH_0}$ is the threshold value determined prior to the first detection cycle of the QRS complex, and $\mathrm{R_0}$ is the maximum value of the ECG signal during one second.

Then, again, using the COMP comparator, the value of the ABS_DIFF_SHORT signal generated at the output of the ABS_DIFF_SHORT_MODULE module is compared with the new threshold TH value determined in this way on the basis of the ECG signal provided by the ECG_MODULE of the ECG measurement module, and the cycle is repeated.

**Claims**

1. Method for detecting a QRS complex of an electrocardiogram (ECG) signal consisting in a reception of the ECG signal obtained by means of an ECG signal measuring module, monitoring the electrical heart activity of the patient, and in a calculation of the difference between the current instantaneous value of the ECG signal and an average value of the ECG signal determined for a time segment of a fixed length wherein an ABS_DIFF_SHORT signal generated at an output of an ABS_DIFF_SHORT_MODULE module is monitored using a COMP comparator, where said ABS_DIFF_SHORT signal is the difference between the current instantaneous value of the ECG signal, provided by an ECG_MODULE measurement module, and an average SHORT_AVG value of the ECG signal calculated for a T_SHORT time segment of a fixed length, then, by means of the COMP comparator, a moment when the ABS_DIFF_SHORT signal reaches a predetermined threshold TH value, determined by a TH_MODULE module, is detected, then, when a moment the ABS_DIFF_SHORT signal reaches the set threshold TH value, a countdown of a SEARCHING_WINDOW time window with a set length for a search for an R wave in the QRS complex of the EKG signal begins using a PULSE GENERATOR pulse generator, then a maximum value of the ABS_DIFF_LONG_MODULE produced on the output of a ABS_DIFF_LONG_MODULE module is recorded during the SEARCHING_WINDOW time window of the ABS_DIFF_LONG signal, using a PEAK_DETECTOR detection module, where said ABS_DIFF_LONG signal being the difference between the current instantaneous value of the ECG signal and a LONG_AVG average value of the ECG signal calculated for a T_LONG time segment of a fixed length and, at the same time, using the PEAK_DETECTOR detection module, the moment of occurrence of the detected maximum value of the ABS_DIFF_LONG signal during the SEARCHING_WINDOW time window is registered, then an R_AMPLITUDE_MEMORY memory module saves the maximum value of the ABS_DIFF_LONG signal, detected by the PEAK_DETECTOR detection module, which is considered to be the R wave amplitude in the QRS complex, and then an R_TIMESTAMP_MEMORY memory module records the moment of occurrence of the maximum value of the/a LONG_ABS_DIFF signal detected by the PEAK_DETECTOR detection module, where said LONG_ABS_DIFF value is considered to be the time of the R wave in the QRS complex, then, using the TH_MODULE module, a new TH threshold value is determined used to measure, using the PULSE_GENERATOR pulse generator, the beginning of the SEARCHING_WINDOW time window in the next cycle of the ECG signal, whereas the new TH threshold value is determined on the basis of the ABS_DIFF_SHORT signal maximum values detected by the PEAK_DETECTOR detection module and stored by the R_AMPLITUDE_MEMORY memory module of maximum values of ABS_DIFF_LONG values in the previous ECG signal cycles, followed by a return to monitoring using the COMP signal comparator of the ABS_DIFF_SHORT signal, generated on the output of the ABS_DIFF_SHORT_MODULE module on the basis of the ECG signal provided by the measuring module of the ECG signal, and then the entire cycle described above is repeated as many times as desired.

2. The method according to claim 1, **characterized in that** the T_SHORT time interval for determining the average value of the SHORT_AVG of the ECG signal is not shorter than 40 ms and also not longer than 100 ms, while the T_LONG time interval for determining the LONG_AVG average value of the ECG signal is not shorter than 150 ms and not longer than 400 ms.

3. The method according to claim 1, **characterized in that** the threshold TH value for the ABS_DIFF_SHORT signal, being the difference between the current instantaneous value of the ECG signal provided by the ECG_MODULE measurement module, and the mean SHORT_AVG value of the ECG signal calculated for the fixed length T_SHORT time interval in a given QRS complex detection cycle is determined by the TH_MODULE module based on the TH value threshold determined by the TH_MODULE module in the previous detection cycle of the QRS complex and the R-wave amplitude in the QRS complex registered in the current detection cycle of the QRS complex recorded with the PEAK_DETECTOR detection module and stored in the R_AMPLITUDE_MEMORY memory module, in such a way that the TH value threshold in a given detection cycle of the QRS complex is the sum of the product of the threshold TH value determined in the previous detection cycle of the QRS complex and the less than one scaling factor and the product of the R-wave amplitude recorded in the current QRS complex detection cycle, one minus the scaling factor, wherein the threshold TH value before the first detection cycle of the QRS complex is determined by the TH_MODULE module in the form of a product of less than one weighting factor and the maximum value of the ECG signal calculated for a time interval of one second.

**Patentansprüche**

1. Verfahren zum Erfassen eines QRS-Komplexes in einem Elektrokardiogramm (EKG)-Signal, das mittels eines EKG-Signal-Messmoduls erhalten wird, das die elektrische Aktivität des Herzens eines Patienten überwacht, wobei das am Ausgang des Moduls ABS_DIFF_SHORT_MODUL erzeugte Signal ABS_DIFF_SHORT mittels eines Komparators COMP überwacht wird und die Differenz zwischen dem aktuellen Momentanwert des vom Messmodul ECG_MODULE gelieferten EKG-Signals und dem Durchschnittswert von SHORT_AVG des EKG-Signals, der über ein Zeitintervall T_SHORT mit festgelegter Länge berechnet wird, darstellt. Daraufhin wird mit Hilfe des Komparators COMP ein Moment, in dem das Signal ABS_DIFF_SHORT einen mit dem Modul TH_MODULE festgelegten Schwellenwert TH erreicht, erfasst und dann, wenn ein Moment, in dem das Signal ABS_DIFF_SHORT den eingestellten Schwellenwert TH erreicht, beginnt ein Countdown eines Zeitfensters SEARCHING_WINDOW mit festgelegter Länge für eine Suche nach einer R-Zacke im QRS-Komplex des EKG-Signals unter Verwendung des Impulsgenerators PULSE GENERATOR, danach wird mit Hilfe des Erfassungsmoduls PEAK_DETECTOR den Höchstwert des am Ausgang des Moduls ABS_DIFF_LONG_MODULE erzeugten Signals ABS_DIFF_ LONG während des Zeitfensters SEARCHING_WINDOW aufgezeichnet, das die Differenz zwischen dem aktuellen Momentanwert des EKG-Signals und einem Mittelwert LONG_AVG des EKG-Signals ist, der über ein Zeitintervall T_LONG mit festgelegter Länge berechnet wird und gleichzeitig wird mit Hilfe des Erfassungsmoduls PEAKJDETECTOR der Zeitpunkt des Auftretens des erfassten Maximalwerts des Signals ABS_DIFF_LONG während des Zeitfensters SEARCHING_WINDOW aufgezeichnet, danach wird mit Hilfe des Speichermoduls R_AMPLITUDE_MEMORY den vom Erfassungsmodul PEAK_DETECTOR erfassten Maximalwert des Signals ABS_DIFF_LONG gespeichert, der als Amplitude der R-Zacke im QRS-Komplex betrachtet wird, und dann wird mit Hilfe des Speichermoduls R_TIMESTAMP_MEMORY der Zeitpunkt des Auftretens des Maximalwerts des Signals LONG_ABS_DIFF gespeichert, der als Zeitpunkt des Auftretens der R-Zacke im QRS-Komplex betrachtet wird, danach wird mit Hilfe des Moduls TH_MODULE ein neuer TH-Schwellenwert bestimmt, der mit Hilfe des Impulsgenerators PULSE_GENERATOR zur Messung des Beginns des Zeitfensters SEARCHING_WINDOW im nächsten Zyklus des EKG-Signals verwendet wird, wobei der neue Schwellenwert TH durch die Maximalwerte des Signals ABS_DIFF_LONG bestimmt wird, die mit dem Erfassungsmodul PEAK_DETECTOR erfasst und mit dem Speichermodul R_AMPLITUDE_MEMORY in den vorhergehenden EKG-Signalzyklen gespeichert wurden, danach wird das Signal ABS_DIFF_SHORT, das am Ausgang des Moduls ABS_DIFF_SHORT_MODULE auf der Grundlage des vom EKG-Signalmessmodul gelieferten EKG-Signals erzeugt wird, mit Hilfe des COMP-Komparators wieder in die Überwachung aufgenommen, und dann wird der gesamte oben beschriebene Zyklus so oft wie gewünscht wiederholt.

2. Verfahren nach Anspruch 1, **dadurch gekennzeichnet, dass** das Zeitintervall T_SHORT, das zur Bestimmung des Mittelwertes SHORT _AVG des EKG-Signals dient, nicht kleiner als 40 ms und gleichzeitig nicht größer als 100 ms ist, während das Zeitintervall T_LONG, das zur Bestimmung des Mittelwertes LONG_AVG des EKG-Signals dient, nicht kleiner als 150 ms und gleichzeitig nicht größer als 400 ms ist.

3. Verfahren nach Anspruch 1, **dadurch gekennzeichnet, dass** der Schwellenwert TH für das Signal ABS_DIFF_SHORT, der die Differenz des aktuellen Momentanwertes des vom Messmodul ECG_MODULE gelieferten EKG-Signals und des Mittelwertes SHORT_AVG des EKG-Signals darstellt, der über das Zeitintervall T_SHORT mit festgelegter Länge berechnet wird, in einem gegebenen Erfassungszyklus des QRS-Komplexes mit Hilfe des Moduls TH_MODULE auf der Grundlage des mit dem Modul TH_MODULE im vorangegangenen Erfassungszyklus des QRS-Komplexes bestimmten Schwellenwertes TH ermittelt wird und auf der Grundlage der Amplitude der im aktuellen QRS-Komplex Erfassungszyklus aufgezeichneten R-Zacke des QRS-Komplexes, die auf die Art und Weise mit dem Erfassungsmodul PEAK_DETECTOR aufgezeichnet und im Speichermodul

R_AMPLITUDE_MEMORY gespeichert wird, so dass der Schwellenwert TH in einem gegebenen Erfassungszyklus des QRS-Komplexes die Summe des Produkts aus dem im vorhergehenden Erfassungszyklus des QRS-Komplexes ermittelten Schwellenwert und einem Skalierungsfaktor kleiner als 1 und des Produkts aus der im aktuellen Erfassungszyklus des QRS-Komplexes aufgezeichneten Amplitude der R-Zocke, einer Eins vermindert um den Skalierungsfaktor sowie einem Gewichtfaktor, der ebenfalls kleiner als 1 ist, ausmacht, wobei der Schwellenwert TH vor dem ersten Erfassungszyklus des QRS-Komplexes mit Hilfe des Moduls TH_MODULE als das Produkt aus dem Gewichtsfaktor, der kleiner als 1 ist, und dem Maximalwert des über ein Zeitintervall von einer Sekunde berechneten EKG-Signals bestimmt wird.

Akademia Górniczo-Hutnicza
im. Stanisława Staszica w Krakowie
BEVOLLMÄCHTIGTER
Maciej Magoński

**Revendications**

1. Méthode de détection d'un complexe QRS d'un signal d'électrocardiogramme (ECG) consistant à recevoir le signal ECG obtenu au moyen d'un module de mesure du signal ECG, à surveiller l'activité cardiaque électrique du patient et à calculer la différence entre la valeur instantanée actuelle du signal ECG et une valeur moyenne du signal ECG déterminée pour un intervalle de temps d'une longueur fixe et ensuite à surveiller un signal ABS_DIFF_SHORT généré à une sortie d'un module ABS_DIFF SHORT_MODULE à l'aide d'un comparateur COMP, où ledit signal ABS_DIFF_SHORT représente la différence entre la valeur instantanée actuelle du signal ECG, fournie par un module de mesure ECG_MODULE, et une valeur moyenne SHORT_AVG du signal ECG calculée pour un intervalle de temps T_SHORT d'une durée fixe; ensuite, à l'aide du comparateur COMP, un moment où le signal ABS_DIFF_SHORT atteint une valeur seuil TH prédéterminée, déterminée par un module TH_MODULE, est détecté, puis, lorsqu'un moment où le signal ABS_DIFF_SHORT atteint la valeur seuil TH fixée, un compte à rebours d'une fenêtre temporelle SEARCHING_WINDOW d'une longueur fixée pour la recherche d'une onde R au sein du complexe QRS du signal ECG commence à l'aide d'un générateur d'impulsions PULSE GENERATOR; ensuite, une valeur maximale du module ABS_DIFF_LONG_MODULE produite sur la sortie d'un module ABS_DIFF_LONG_MODULE est enregistrée pendant la fenêtre de temps SEARCHING_WINDOW du signal ABS_DIFF_LONG, à l'aide d'un module de détection PEAK_DETECTOR, ledit signal ABS_DIFF_LONG représentant la différence entre la valeur instantanée actuelle du signal ECG et une valeur moyenne LONG_AVG du signal ECG calculée pour un intervalle de temps T_LONG d'une longueur fixe, et, en même temps, à l'aide du module de détection PEAK_DETECTOR, le moment de l'occurrence de la valeur maximale détectée du signal ABS_DIFF_LONG pendant la fenêtre de temps SEARCHING_WINDOW est enregistré, puis un module de mémoire R_AMPLITUDE_MEMORY enregistre la valeur maximale du signal ABS_DIFF_LONG, détectée par le module de détection PEAK_DETECTOR, qui est considérée comme l'amplitude de l'onde R au sein du complexe QRS, et ensuite un module de mémoire R TIMESTAMP_MEMORY enregistre le moment de l'occurrence de la valeur maximale du / d'un signal LONG_ABS_DIFF détecté par le module de détection PEAK_DETECTOR, où ladite valeur LONG_ABS_DIFF est considérée comme le temps de l'onde R au sein du complexe QRS, puis, à l'aide du module TH_MODULE, une nouvelle valeur seuil TH est déterminée et utilisée pour mesurer, à l'aide du générateur d'impulsions PULSE_GENERATOR, le début de la fenêtre temporelle SEARCHING_WINDOW dans le cycle suivant du signal ECG, alors que la nouvelle valeur seuil TH est déterminée sur la base des valeurs maximales du signal ABS_DIFF_SHORT détectées par le module de détection PEAK_DETECTOR et stockées par le module de mémoire R_AMPLITUDE_MEMORY des valeurs maximales ABS_DIFF_LONG dans les cycles précédents du signal ECG, suivi d'un retour à la surveillance à l'aide du comparateur de signal COMP du signal ABS_DIFF_SHORT, généré en sortie du module ABS_DIFF SHORT_MODULE sur la base du signal ECG fourni par le module de mesure du signal ECG; ensuite, l'ensemble du cycle décrit ci-dessus est répété autant de fois que souhaité.

2. La méthode selon la revendication 1, **caractérisée en ce que** l'intervalle de temps T_SHORT utilisé pour déterminer la valeur moyenne SHORT_AVG du signal ECG n'est pas inférieur à 40 ms ni supérieur à 100 ms, tandis que l'intervalle de temps T_LONG utilisé pour déterminer la valeur moyenne LONG_AVG du signal ECG n'est pas inférieur à 150 ms ni supérieur à 400 ms.

3. La méthode selon la revendication 1, **caractérisée en ce que** la valeur seuil TH pour le signal ABS_DIFF_SHORT, représentant la différence entre la valeur instantanée actuelle du signal ECG fournie par le module de mesure ECG_MODULE, et la valeur moyenne SHORT_AVG du signal ECG calculée pour l'intervalle de temps de longueur fixe T_SHORT dans un cycle de détection du complexe QRS donné, est déterminée par le module TH_MODULE sur la base du seuil de la valeur TH déterminé par le module TH_MODULE dans le cycle de détection précédent du complexe QRS et de l'amplitude de l'onde R au sein du complexe QRS relevée dans le cycle de détection actuel du complexe QRS enregistré par le module de détection PEAK_DETECTOR et stockée dans le module de mémoire R_AMPLITUDE_MEMORY, de sorte que le seuil de la valeur TH dans un cycle de détection donné du complexe QRS soit la somme du produit de la valeur TH seuil déterminée dans le cycle de détection précédent du complexe QRS et du facteur d'échelle inférieur à un et du produit de l'amplitude de l'onde R enregistrée dans le cycle de détection actuel du complexe QRS, un moins le facteur d'échelle, la valeur seuil TH avant le premier cycle de détection du complexe QRS étant déterminée par le module TH_MODULE sous la forme d'un produit du facteur de pondération inférieur à un et de la valeur maximale du signal ECG calculée pour un intervalle de temps d'une seconde.

# EP 3 970 615 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5738104 A
- US 5188116 A
- US 9414761 B
- KR 20160107390

**Non-patent literature cited in the description**

- **J. PAN** ; **W.J. TOMPKINS**. A Real-Time QRS Detection Algorithm. *IEEE Transactions on Biomedical Engineering*, 1985, vol. 32 (3)
- **CHEN, H. C.** ; **CHEN, S. W.** A moving average based filtering system with its application to real-time QRS detection. *Computers in Cardiology*, 2003, 585-588